# EUROPÄISCHE PATENTANMELDUNG

(11) **EP 2 548 536 A1**
(43) Veröffentlichungstag der Anmeldung: **23.01.2013**
(21) Anmeldenummer: 11174959.4
(22) Anmeldetag: 22.07.2011
(51) Int. Cl.: A61F 7/03, A47C 7/74

(54) **Verwendung eines mit einer unterkühlbaren Lösung gefüllten Wärmekissens**

(71) Anmelder: Dipl.-Ing. Dr. Ernst Vogelsang GmbH & Co. KG, D-45699 Herten (DE)
(72) Erfinder: Vogelsang, Dirk, 45657 Recklinghausen (DE)
(74) Vertreter: Nunnenkamp, Jörg

(57) **Zusammenfassung**

Gegenstand der vorliegenden Erfindung ist die Verwendung eines mit einer unterkühlbaren Lösung gefüllten Wärmekissens (1) als Sitzauflage, insbesondere zum Einsatz bei Freiluftveranstaltungen. Das Wärmekissen (1) ist mit einer Hülle (4a, 4b), und wenigstens einer Kammer (5, 6) in der Hülle (4a, 4b), ausgerüstet. Erfindungsgemäß sind zwei voneinander separierte und mit jeweils einem zugehörigen Auslöser (8) ausgerüstete Kammern (5, 6) im Innern der Hülle (4a, 4b) vorgesehen, so dass deren jeweilige Füllungen getrennt voneinander aktivierbar sind.

## Beschreibung

Die Erfindung betrifft die Verwendung eines mit einer unterkühlbaren Lösung gefüllten Wärmekissens als Sitzauflage, insbesondere zum Einsatz bei Freiluftveranstaltungen, mit einer Hülle, und mit wenigstens einer Kammer in der Hülle.

Wärmekissen des zuvor angegebenen Aufbaus sind allgemein bekannt und beispielsweise mit einer Lösung auf Basis von Natriumacetat gefüllt. Der unterkühlte Zustand dieser Lösung kann durch mechanischen Energieeintrag mittels bspw. eines Metallplättchens oder unter Rückgriff auf einen vergleichbaren Auslöser verändert und dadurch die Kristallisation der Lösung ausgelöst werden. Da die Kristallisation in einem exothermen Prozess abläuft, werden Temperaturen von mehr als 50°C beim Übergang der unterkühlten Lösung vom flüssigen in den festen Zustand beobachtet.

Als Ursache für die Kristallisation kann regelmäßig eine Druckwelle verantwortlich gemacht werden, die durch das Drücken des Metallplättchens oder eines anderen Auslösers in der übersättigten Lösung ausgelöst wird. Dadurch kommt es zur Freisetzung von mikroskopisch kleinen Kristallisationskeimen. Beim beobachteten Phasenübergang flüssig-fest wird die zuvor eingebrachte Schmelzwärme frei und sorgt für die beschriebene Erwärmung.

Um das Wärmekissen wieder "aufzuladen" wird im Beispielfall das Natriumacetat oder werden vergleichbare Salze oder Paraffine als Speichermedium geschmolzen. Die genannten Salze oder Paraffine nehmen hierbei sehr viel Wärmeenergie als Schmelzwärme auf. Da der Vorgang reversibel ist, kann die zuvor aufgenommene Wärmemenge beim Erstarren, d. h. beim mit Hilfe des Auslösers initiierten Übergang flüssig-fest wieder abgegeben werden. Diese grundsätzlichen Funktionsweisen und Zusammenhänge sind bekannt, wozu nur beispielhaft auf die DE 100 58 642 A1 verwiesen sei.

Hier geht es um ein Wärmekissen, in dessen Hülle ein metallischer Körper so gelagert ist, dass er bei einer elastischen Verformung der Hülle eine derartige elastische Verformung erfährt, dass dadurch die Kristallisation der Lösung in einem exothermen Prozess ausgelöst wird. Zu diesem Zweck besteht der metallische Körper aus einem einzelnen, in sich gebogenen Teil. Die Hülle ist als Kunststoffüberzug ausgelegt.

Eine gattungsgemäße Verwendung ist durch die Gebrauchsmusterschrift DE 20 2007 003 866 U1 bekannt geworden. Hier geht es um ein Wärmekissen, welches aus einem Kunststoffbeutel besteht, der mit der unterkühlbaren Lösung gefüllt ist. Außerdem ist ein Aktivierungselement bzw. ein Auslöser realisiert. Der Kunststoffbeutel verfügt über wenigstens eine durch Verschweißen der Kunststofflagen abgegrenzte und über Kanäle mit dem übrigen Kunststoffbeutel verbundene Kammer. In der Kammer ist das Aktivierungselement schwimmend gelagert, wobei die Kanäle so angeordnet sind, dass das Aktivierungselement daran gehindert wird, in den übrigen Kunststoffbeutel zu schwimmen. Auf diese Weise erfährt das Aktivierungselement bzw. der Auslöser keine Behinderung und wird eine leichte Bedienung ermöglicht. Dadurch lässt sich das Wärmekissen einfach falten, und zwar ohne dass das Aktivierungselement bzw. der Auslöser behindert wird. Das bekannte Wärmekissen kann in einer textilen Umhängetasche transportiert werden und lässt sich beispielsweise in Stadien als Sitzkissen bzw. Sitzauflage nutzen.

Zum weiteren relevanten Stand der Technik gehört darüber hinaus ein Wärmekissen, wie es in dem Gebrauchsmuster DE 91 13 556 U1 beschrieben wird. Das fragliche Wärmekissen wird für therapeutische Anwendungen genutzt und beispielsweise auf Nieren-, Rücken-, Nacken-, Gelenk- oder Muskelpartien aufgelegt, um dort entstehende oder vorhandene Schmerzen zu lindern. Dabei soll insgesamt eine verbesserte therapeutische Wirkung erzielt werden. Zu diesem Zweck sind im Innern der Hülle mehrere voneinander getrennte Kammern mit der unterkühlbaren Lösung vorgesehen. Jede Kammer weist einen Auslöser auf.

Der Erfindung liegt das technische Problem zugrunde, die gattungsgemäße Verwendung eines mit einer unterkühlbaren Lösung gefüllten Wärmekissens als Sitzauflage so zu verbessern, dass auch längere Aufenthaltsdauern im Freien problemlos selbst bei tiefen oder sogar negativen Temperaturen überbrückt werden können.

Zur Lösung dieser technischen Problemstellung ist die gattungsgemäße Verwendung im Rahmen der Erfindung dadurch gekennzeichnet, dass im Innern der Hülle wenigstens zwei voneinander separierte und mit jeweils einem zugehörigen Auslöser ausgerüstete Kammern vorgesehen sind, so dass deren jeweilige Füllungen mit der unterkühlbaren Lösung getrennt voneinander aktivierbar sind.

Grundsätzlich sind zwei und mehr Kammern im Innern der Hülle mit zugehörigem Auslöser durch die DE 91 13 556 U1 bekannt geworden. Hier stehen allerdings therapeutische Anwendungen im Vordergrund und kommt es darauf an, die Größe des Kissens nach dem jeweiligen Anwendungszweck auszurichten. Denn entsprechend der Größe des Kissens kann eine zugehörige Körperpartie mit Wärme beaufschlagt werden.

Im Rahmen der Erfindung geht es jedoch darum, eine möglichst lange Heizwirkung der Sitzauflage besonders beim Einsatz im Zusammenhang mit Freiluftveranstaltungen zu gewährleisten. Zu diesem Zweck behält die Sitzauflage ausdrücklich ihre Größe bei und ist formstabil ausgelegt. Außerdem werden die beiden Kammern vorteilhaft miteinander verschränkt bzw. sind ineinandergeschachtelt. D. h., die wenigstens zwei Kammern greifen meistens ineinander, und zwar in ihrer Flächenausdehnung bzw. in Aufsicht auf das Wärmekissen.

Die einzelnen Kammern können zeitlich versetzt wahlweise ausgelöst werden. Das wird ein Bediener je nach den äußeren Freiluftbedingungen und im Bedarfsfall selbst vornehmen. Um das erfindungsgemäße Wärmekissen nach Gebrauch wieder in seinen ursprünglichen Zustand zurückzuversetzen, wird dieses beispielsweise in ein kochendes Wasserbad eingetaucht. Auf diese Weise kann die auskristallisierte Lösung wieder in ihren unterkühlten (flüssigen) Zustand zurückversetzt werden, und zwar jede einzelne der wenigstens zwei voneinander separierten Kammern.

Nach vorteilhafter Ausgestaltung sind die Kammern durch wenigstens eine Trennwand voneinander getrennt. Im Regelfall verbindet die Trennwand eine obere und eine untere Materialbahn der Hülle miteinander. Meistens setzt sich die Hülle aus der oberen und der unteren Materialbahn zusammen, die randseitig miteinander verbunden, beispielsweise miteinander verschweißt, sind. Denn als geeigneter Werkstoff für die Materialbahn hat sich Kunststoff als besonders günstig erwiesen und hier insbesondere ein thermoplastischer Kunststoff wie beispielsweise PE (Polyethylen), PP (Polypropylen) etc.. Im Regelfall sind beide Materialbahnen aus einem übereinstimmenden thermoplastischem Kunststoff hergestellt, der darüber hinaus noch vorteilhaft transparent gestaltet sein mag.

Auf diese Weise kann vom Bediener insgesamt der Kristallisationsvorgang und folglich die richtige Funktionsweise des Wärmekissens unschwer beobachtet werden. - Die zuvor bereits angesprochene Trennwand ist im Regelfall als Schweißverbindungswand und insbesondere Kunststoffschweißverbindungswand ausgebildet. Tatsächlich werden die beiden Materialbahnen im Bereich der Trennwand durch eine Kunststoffschweißung miteinander verbunden, so dass sich auf diese Art und Weise die Trennwand bzw. Schweißverbindungswand automatisch bildet.

Im Regelfall ist die Trennwand mäanderförmig geschlungen. Dadurch können die wenigstens zwei Kammern als jeweils Fingerkammern ausgebildet werden. Hier hat es sich bewährt, wenn die beiden Fingerkammern gegenüberliegend ausgerichtet sind und ineinander greifen.

Im Übrigen wird man die Auslegung meistens so treffen, dass die beiden Kammern mit jeweils übereinstimmenden Volumen ausgerüstet sind. Das gelingt regelmäßig derart, dass die beiden Kammern in Aufsicht auf die Hülle eine im Wesentlichen übereinstimmende Flächenbelegung besitzen. D. h., die Kammern stimmen üblicherweise hinsichtlich ihrer Flächenbelegung im Bezug auf die Hülle und auch im Hinblick auf ihr Volumen für die Aufnahme der unterkühlbaren Lösung überein.

Außerdem ist die Auslegung meistens so getroffen, dass die beiden Kammern eine Flächenineinanderschachtelung derart aufweisen, dass jede Teilfläche vorgegebener Größe als Bestandteil einer Gesamtfläche der Sitzauflage eine größtenteils übereinstimmende Flächenbelegung von einerseits der einen Kammer und andererseits der anderen Kammer aufweist.

Diese Teilfläche vorgegebener Größe ist in Folge des beschriebenen Einsatzes des erfindungsgemäßen Wärmekissens als Sitzauflage typischerweise an ein (kleines) menschliches Gesäß angepasst und im Bereich von ca. 25 cm² und mehr angesiedelt. D. h., jede Teilfläche von 25 cm² oder mehr der Gesamtfläche der Sitzauflage verfügt durchweg über eine übereinstimmende Flächenbelegung von einerseits der einen Kammer und andererseits der anderen Kammer. Das gilt unabhängig davon, wo sich die Teilfläche im Vergleich zur Gesamtfläche befindet. Egal, an welcher Stelle sich folglich ein Bediener auf das Wärmekissen setzt, ist sichergestellt, dass die fragliche Teilfläche von beiden Kammern in etwa gleich beaufschlagt wird und auch beaufschlagt werden kann. Die von den getrennten Kammern erzeugten Wärmemengen innerhalb dieser Teilfläche stimmt also überein.

Auf diese Weise kann die Zeit der Wärmeabgabe im Vergleich zu einer Ausführungsform mit nur einen Kammer praktisch verdoppelt werden. Denn die fragliche Teilfläche von wenigstens 25 cm² Größe oder mehr wird immer von beiden Kammern belegt, und zwar mit überwiegend übereinstimmender Flächenbelegung. Wenn also die eine Kammer auskristallisiert worden ist und ihre Wärme insgesamt abgegeben hat, kann danach die andere benachbarte Kammer ausgelöst werden und für eine fortgesetzte Wärmeabgabe sorgen. Die Wärmeabgabezeit lässt sich hierdurch theoretisch und praktisch verdoppeln.

Wie bereits erläutert, setzt sich die Hülle aus der oberen und der unteren Materialbahn zusammen, die im Randbereich miteinander verschweißt sind. Dabei werden die Schweißverbindung im Randbereich und die Schweißverbindung zur Herstellung der Trennwand im Allgemeinen zeitgleich und in einem Zug vorgenommen. Auf diese Weise lassen sich die Herstellungskosten besonders günstig einstellen.

Im Übrigen kann das erfindungsgemäße Wärmekissen mit einem umhüllenden Bezug aus beispielsweise einem Textilmaterial und/oder einem Schaumstoff ausgerüstet werden. Bei dem Bezug aus dem Textilmaterial mag es sich um einen Sitzbezug handeln. Der umhüllende Bezug aus Schaumstoff bzw. ein entsprechend gestaltetes Schaumstoffkissen mit Einschubschlitz für das Wärmekissen kann von seiner Größe und Gestaltung her an eine Sitzschale oder einen Sitz angepasst werden, wie sie bei Freiluftveranstaltungen üblicherweise vorhanden sind und von Zuschauern genutzt werden.

Um an dieser Stelle die Wärmeisolation und Wärmeabgabe primär zum Gesäß des Benutzers hin zu steigern, hat sich bewährt, wenn eine Unterseite des Wärmekissens mit einer wärmeisolierenden Auflage ausgerüstet ist. Diese wärmeisolierende Auflage kann grundsätzlich aus zwei Schichten aufgebaut sein, nämlich einer wärmeisolierenden Schicht dem Sitzmöbel zugewandt und einer wärmereflektierenden Schicht dem Wärmekissen zugewandt. Die wärmeisolierende Schicht stellt sicher, dass die vom Wärmekissen erzeugte Wärme nicht an die Sitzschale oder das Sitzmöbel abgegeben wird. Mit der wärmereflektierenden Schicht wird erreicht, dass etwaige Wärmestrahlung von der Lösung in Richtung auf den Bediener zurückreflektiert wird. Demgegenüber wird man die Oberseite des umhüllenden Bezuges für das Wärmekissen entweder mit keiner oder mit einer lediglich dünnen Auflage versehen, um den Wärmetransport vom Wärmekissen durch die Auflage hindurch bis zum Bediener bzw. dessen Gesäß hin möglichst günstig zu gestalten. Hier kann sogar mit einer wärmeleitenden Auflage gearbeitet werden, beispielsweise einer Metallfolie.

Im Ergebnis wird die neuartige Verwendung eines speziell ausgelegten Wärmekissens als Sitzauflage beschrieben, die sich insbesondere zum Einsatz bei Freiluftveranstaltungen wie beispielsweise Sportveranstaltungen besonders eignet. Zu nennen sind hier insbesondere Fußballveranstaltungen aber auch Motorsportveranstaltungen, Konzerte etc.. Um an dieser Stelle eine möglichst langandauernde und gleichmäßige Wärmeabgabe des Wärmekissens zur Verfügung zu stellen, ist seine Hülle mit zumindest zwei voneinander separierten und mit jeweils einem zugehörigen Auslöser ausgerüsteten Kammern aufgebaut. Die einzelnen Kammern sind so flächig ineinander geschachtelt, dass jede Teilfläche vorgegebener Größe von beispielsweise 25 cm² und mehr, als Bestandteil der Gesamtfläche der Sitzauflage in jedem Fall eine übereinstimmende Flächenbelegung von einerseits der einen Kammer und andererseits der anderen Kammer aufweist.

D. h., egal an welcher Stelle der Gesamtfläche der Sitzauflage sich die besagte Teilfläche befindet, ist die betreffende Bemessungsregel gewährleistet. Dadurch wird eine im Vergleich zu einer Ausführungsform mit nur einer einzigen Kammer quasi doppelte Wärmeabgabezeit beobachtet. Denn die beiden Kammern geben unabhängig voneinander eine aufgrund der übereinstimmenden Flächenbelegung innerhalb der Teilfläche gleiche oder praktisch vergleichbare Wärmemenge ab. Dadurch kann zunächst die eine Kammer verbraucht werden und lässt sich erst im Anschluss hieran die andere Kammer auslösen und aktivieren.

Auf diese Weise kommt es zu einer signifikanten Verlängerung der Wärmeabgabezeit und zu bisher nicht beobachteten Komfortverbesserungen. Denn wenn man beispielsweise davon ausgeht, dass die Wärmeabgabezeit bei einer Kammer typischerweise bis zu ca. einer Stunde je nach den herrschenden Außentemperaturen betragen kann, lassen sich durch die erfindungsgemäße Auslegung insgesamt Wärmeabgabezeiten von bis zu 2 Stunden oder noch mehr realisieren, was insbesondere für den Einsatz bei Freiluftveranstaltungen besonders vorteilhaft ist und den Bedienungskomfort erheblich verbessert.

Hinzu kommt, dass das beschriebene Wärmekissen problemlos entsorgt werden kann und unter Umweltgesichtspunkten völlig unproblematisch ist. Denn die meistens eingesetzte Lösung aus Natriumacetat ist allgemein lebensmittelverträglich, so dass etwaige Gefährdungen beim Austreten der Lösung von vornherein ausgeschlossen werden können. Da die Hülle üblicherweise aus thermoplastischem Kunststoff wie beispielsweise PE oder PP hergestellt ist, kann sie - ohne die Lösung - problemlos einem Recyclingkreislauf zugeführt werden. Im Übrigen lassen sich derartige Hüllen besonders einfach verarbeiten, weil die beschriebenen Kunststoffe relativ niedrige Schmelztemperaturen von nicht viel mehr als 100°C aufweisen, so dass die Schweißverbindungen problemlos zu realisieren sind. Die Größe des Wärmekissens insgesamt beträgt typischerweise 500 cm² und mehr. Das hängt von dem Bediener, der Größe des als Unterlage fungierenden Sitzmöbels, der Sitzschale etc. ab.

Im Folgenden wird die Erfindung anhand einer lediglich ein Ausführungsbeispiel darstellenden Zeichnung näher erläutert; es zeigen:
- Fig. 1: eine Aufsicht auf ein erfindungsgemäßes Wärmekissen in einer Übersicht und
- Fig. 2: einen Schnitt durch das als Sitzauflage ausgeführte Wärmekissen ebenfalls schematisch.

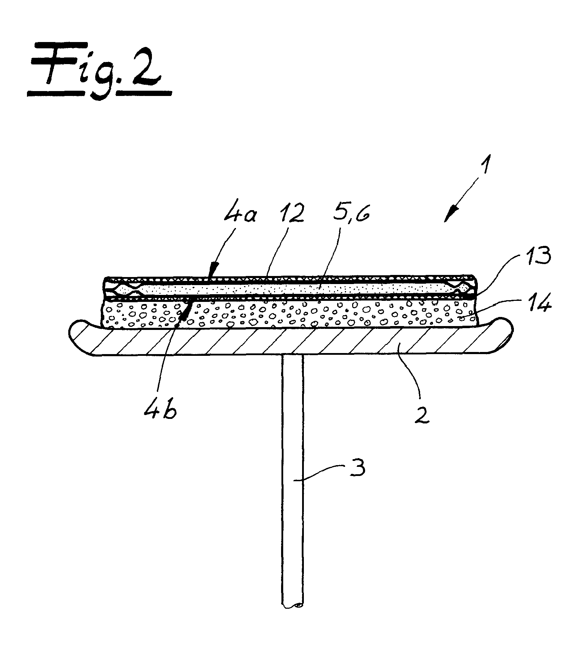

In den Figuren ist ein Wärmekissen 1 dargestellt, welches ausweislich der Fig. 2 als Sitzauflage Verwendung findet. Tatsächlich wird das Wärmekissen 1 auf ein Sitzmöbel 2 aufgelegt. Bei dem Sitzmöbel 2 handelt es sich im gezeigten Beispielfall um eine Sitzschale 2 aus Kunststoff, wie sie beispielsweise in Fußballstadien Verwendung findet. Derartige Sitzschalen 2 werden auf Säulen 3 abnehmbar montiert. Die Sitzschale 2 verfügt über eine im Querschnitt leicht konvex nach oben gewölbte Oberfläche, deren Ränder also geringfügig höher als ein mittlerer Bereich ausgebildet sind.

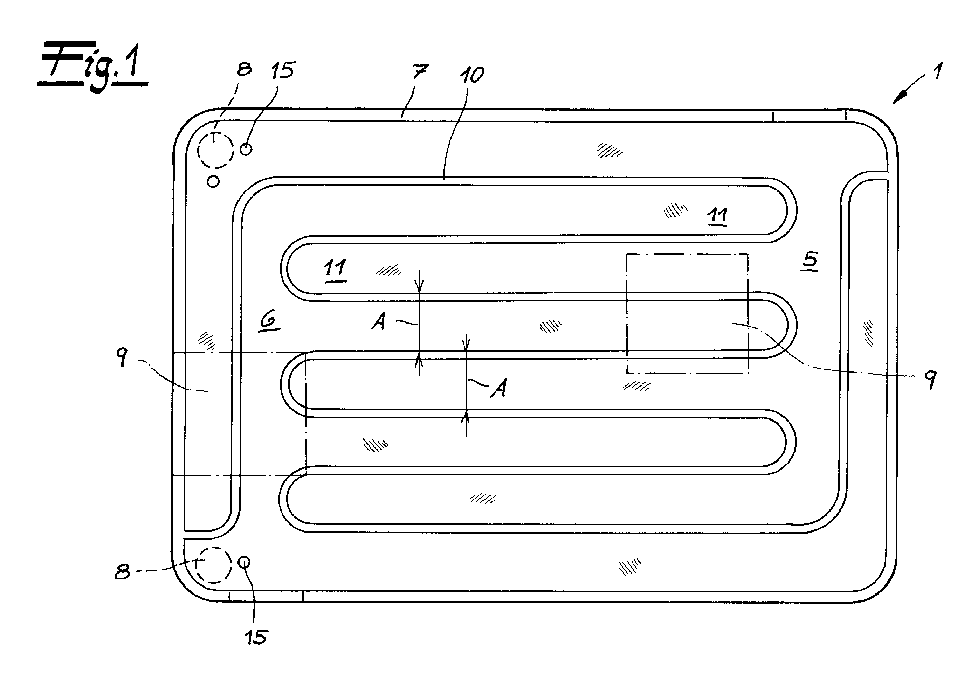

Das als Sitzauflage fungierende Wärmekissen 1 kommt insbesondere bei Freiluftveranstaltungen und hier Fußballspielen, Konzerten, Motorsportveranstaltungen etc. zum Einsatz. Zu diesem Zweck setzt sich das Wärmekissen 1 ausweislich der Darstellung nach Fig. 1 aus einer Hülle 4a, 4b und wenigstens einer Kammer 5, 6 in der Hülle 4a, 4b zusammen. Die Hülle 4a, 4b besteht aus einer oberen Materialbahn 4a und einer unteren Materialbahn 4b, die im Randbereich 7 miteinander verbunden, nach dem Ausführungsbeispiel miteinander verschweißt, sind. Zu diesem Zweck ist im Randbereich 7 eine umlaufende Schweißnaht realisiert, welche die beiden Materialbahnen 4a, 4b in der Art eines geschlossenen Beutels miteinander verbindet.

Man erkennt, dass zwei voneinander separierte und mit jeweils einem zugehörigen Auslöser 8 ausgerüstete Kammern 5, 6 im Innern der Hülle 4a, 4b vorgesehen sind. Bei den beiden Auslösern 8 handelt es sich im Ausführungsbeispiel jeweils um ein knickbares Metallplättchen, welches durch einen Knickvorgang die Kristallisation des zuvor flüssigen und im Innern der jeweiligen Kammern 5, 6 befindlichen Natriumacetates als unterkühlbare Lösung initiiert. Die Kristallisation der unterkühlbaren Lösung führt wie beschrieben dazu, dass beim Kristallisationsvorgang Wärme abgegeben wird.

Die separierten Kammern 5, 6 sind jeweils mit der fraglichen unterkühlbaren Lösung gefüllt und lassen sich getrennt voneinander aktivieren. Den jeder Kammer 5, 6 ist jeweils ein eigener Auslöser 8 zugeordnet. Man erkennt anhand der Darstellung in Fig. 1, dass die beiden Auslöser 8 an einer Randseite des rechteckförmigen Wärmekissens 1 angeordnet sind. Tatsächlich finden sich beide Auslöser 8 an einer Schmalrandseite des rechteckförmigen Wärmekissens 1. Auf diese Weise können die beiden Auslöser 8 unschwer von einem auf dem Wärmekissen 1 sitzenden Bediener beaufschlagt werden.

Das Wärmekissen 1 weist im dargestellten Beispielfall eine Fläche von ca. 500 cm² und mehr auf. Dadurch wird das Gesäß eines Bedieners beim Sitzen auf der solchermaßen ausgelegten Sitzauflage entsprechend der Darstellung nach Fig. 2 praktisch vollflächig gewärmt, und zwar durch die bei dem beschriebenen Kristallisationsvorgang frei werdende exotherme Wärme. Tatsächlich sind die beiden Kammern 5, 6 hinsichtlich ihrer Flächenbelegung ineinander geschachtelt. Diese Flächenineinanderschachtelung der beiden Kammern 5, 6 ist so gestaltet, dass jede Teilfläche 9 vorgegebener Größe als Bestandteil der Gesamtfläche der Sitzauflage bzw. des Wärmekissens 1 eine größtenteils übereinstimmende Flächenbelegung von einerseits der einen Kammer 5 und andererseits der anderen Kammer 6 aufweist.

Die fragliche Teilfläche 9 verfügt über eine Größe von üblicherweise 25 cm² und mehr. Im Rahmen des Ausführungsbeispiels kann die Gesamtfläche folglich in wenigstens zehn, nach dem gezeigten Beispiel in zwanzig oder noch mehr der besagten Teilflächen 9 unterteilt werden, die jeweils aneinander anschließen. Das ist selbstverständlich nur beispielhaft zu verstehen. Von besonderer Bedeutung ist jedenfalls der Umstand, dass innerhalb der jeweiligen Teilfläche 9 die von der ersten Kammer 5 belegte Fläche in etwa derjenigen Fläche entspricht, die von der zweiten Kammer 6 belegt wird.

Dadurch wird eine gleichmäßige und lang andauernde Wärmeabgabe zur Verfügung gestellt. Denn nachdem beispielsweise die erste Kammer 5 aktiviert worden ist und ihre Wärme abgegeben hat, kann die zweite Kammer 6 ausgelöst werden. Durch die übereinstimmende oder nahezu übereinstimmende Flächenbelegung innerhalb der bzw. jeder Teilfläche 9 wird gewährleistet, dass der Bediener eine vergleichbare Wärmeentwicklung über die gesamte Wärmeabgabezeit beobachtet. Durch den Rückgriff auf zwei oder mehr Kammern 5, 6 ist dabei insgesamt die Wärmeabgabezeit im Vergleich zur Ausführungsform mit nur einen Kammer wenigstens verdoppelt.

Das wird im Detail so erreicht, dass die beiden Kammern 5, 6 durch wenigstens eine Trennwand 10 voneinander getrennt sind. Bei der Trennwand 10 handelt es sich um eine Schweißverbindungswand 10 und nach dem Ausführungsbeispiel eine Kunststoffschweißverbindungswand 10. Denn die beiden Materialbahnen 4a, 4b sind jeweils aus einem thermoplastischen Kunststoff wie PE bzw. PP hergestellt. Die Trennwand 10 wird dabei ebenso wie die umlaufende Schweißnaht im Randbereich 7 durch einen Kunststoffschweißvorgang definiert, bei welchem die beiden Hüllen 4a, 4b miteinander verbunden werden. Die Trennwand 10 und auch die randseitige Schweißverbindung werden meistens in einem Zug und einem übereinstimmenden Herstellungsvorgang produziert, um die Kosten möglichst gering zu halten.

Mit Hilfe der Trennwand 10 und natürlich auch der randseitigen Schweißverbindung werden die obere Materialbahn 4a und die untere Materialbahn 4b der Hülle 4a, 4b miteinander verbunden. Man erkennt, dass die im Innern der Hülle 4a, 4b verlaufende Trennwand 10 mäanderförmig geschlungen ist. Dadurch sind die beiden Kammern 5, 6 als jeweils Fingerkammern 5, 6 ausgebildet.

Die Fig. 1 macht deutlich, dass sich die beiden Fingerkammern 5, 6 gegenüberliegen und ineinandergreifen. Die beiden Kammern 5, 6 sind mit jeweils einem übereinstimmenden Volumen an der unterkühlbaren Lösung gefüllt. Tatsächlich verfügen die beiden Kammern 5, 6 in Aufsicht auf die Hülle 4a, 4b über eine im Wesentlichen übereinstimmende Flächenbelegung.

Außerdem sind die Kammern 5, 6 im Querschnitt kissenförmig gestaltet, wie die Fig. 2 andeutet. Die einzelnen Finger 11 der beiden Fingerkammern 5, 6 besitzen eine jeweils übereinstimmende Breite A. Das gilt auch im Randbereich. Dadurch kann die bereits angesprochene Teilfläche 9 praktisch überall im Vergleich zur vom Wärmekissen 1 abgedeckten Fläche platziert werden, wobei die fragliche Teilfläche 9 dann über die bereits angesprochene übereinstimmende Flächenbelegung von einerseits der ersten Kammer 5 und andererseits der zweiten Kammer 6 verfügt.

Das Wärmekissen 1 ist im Regelfall transparent ausgebildet. Als Werkstoff für die obere Materialbahn 4a und die untere Materialbahn 4b hat sich jeweils übereinstimmend ein thermoplastischer Kunststoff wie beispielsweise PE, PP etc. als besonders günstig erwiesen. Außerdem kann das gesamte Wärmekissen 1 mit einem umhüllenden Bezug 12, 13, 14 ausgerüstet sein. Bei diesem Bezug 12, 13, 14 mag es sich um eine aus einem textilen Material hergestellte Tasche handeln. Alternativ oder zusätzlich kann auch ein Bezug 12, 13, 14 aus Schaumstoff realisiert werden.

Im Rahmen des Ausführungsbeispiels ist die Unterseite des Wärmekissens 1 mit einer wärmeisolierenden Auflage 13, 14 ausgerüstet. Diese wärmeisolierenden Auflage 13, 14 setzt sich aus einer wärmeisolierenden Schicht 14 sitzmöbelseitig und einer wärmereflektierenden Schicht 13 kissenseitig zusammen. Die wärmeisolierende Schicht 14 mag als Schaumstoffschicht oder vergleichbare Kunststoffschicht ausgebildet werden. Bei der wärmereflektierenden Schicht 13 kann es sich um eine Metallfolie, beispielsweise eine Aluminiumfolie handeln.

Die obere Auflage 12 ist meistens dünn gestaltet. Auch an dieser Stelle kann eine Metallfolie zum Einsatz kommen, die eine besonders gute Wärmeleitung vom Wärmekissen 1 zum Bediener bzw. dessen Gesäß zur Verfügung stellt. Der gesamte umhüllende Bezug 12, 13, 14 lässt sich zugleich als Tragetasche für das Wärmekissen 1 verwenden. Insofern können problemlos Aufdrucke, Vereinsembleme etc. aufgebracht werden, mit deren Hilfe eine Identifizierung möglich ist bzw. die Vereinszugehörigkeit dokumentiert werden kann.

Schlussendlich erkennt man in der Fig. 1 einzelne Schweißpunkte bzw. Verbindungspunkte 15. Im Bereich des jeweiligen Schweißpunktes 15 sind die beiden Materialbahnen 4a, 4b - ähnlich wie bei der Trennwand 10 und auch der Schweißverbindung im Randbereich 7 - miteinander verbunden. Mit Hilfe des einen bzw. der beiden Schweißpunke 15 wird die Position des jeweiligen Auslösers 8 festgelegt, und zwar an der bereits genannten Randseite des rechteckförmigen Wärmekissens 1, und zwar in der jeweils zugehörigen Kammer 5, 6. Bei der Randseite handelt es sich um die Schmalrandseite des rechteckförmigen Wärmekissens 1.

Der eine bzw. die mehreren Schweißpunkte respektive Verbindungspunkte 15 werden in einem Arbeitsgang zusammen mit der Trennwand 10 und der Schweißverbindung der beiden Materialbahnen 4a, 4b im Randbereich 7 hergestellt. Dadurch können die Produktionskosten für das Wärmekissen 1 besonders niedrig gehalten werden. Außerdem ermöglicht die Platzierung der jeweiligen Auslöser 8 an der fraglichen Schmalrandseite eine besonders einfache Bedienung.

## Patentansprüche

1. Verwendung eines mit einer unterkühlbaren Lösung gefüllten Wärmekissens (1) als Sitzauflage, insbesondere zum Einsatz bei Freiluftveranstaltungen, mit einer Hülle (4a, 4b), und mit wenigstens einer Kammer (5, 6) in der Hülle (4a, 4b), **dadurch gekennzeichnet, dass** wenigstens zwei voneinander separierte und mit jeweils einem zugehörigen Auslöser (8) ausgerüstete Kammern (5, 6) im Innern der Hülle (4a, 4b) vorgesehen sind, so dass deren jeweilige Füllungen getrennt voneinander aktivierbar sind.

2. Verwendung nach Anspruch 1, **dadurch gekennzeichnet, dass** die Kammern (5, 6) durch wenigstens eine Trennwand (10) voneinander getrennt sind.

3. Verwendung nach Anspruch 2, **dadurch gekennzeichnet, dass** die Trennwand (10) eine obere Materialbahn (4a) und eine untere Materialbahn (4b) der Hülle (4a, 4b) miteinander verbindet.

4. Verwendung nach Anspruch 2 oder 3, **dadurch gekennzeichnet, dass** die Trennwand (10) als Schweißverbindungswand (10), insbesondere Kunststoffschweißverbindungswand (10), ausgebildet ist.

5. Verwendung nach einem der Ansprüche 2 bis 4, **dadurch gekennzeichnet, dass** die Trennwand (10) mäanderförmig geschlungen ist, so dass die beiden Kammern (5, 6) als jeweils Fingerkammern (5, 6) ausgebildet sind.

6. Verwendung nach Anspruch 5, **dadurch gekennzeichnet, dass** sich die beiden Fingerkammern (5, 6) gegenüberliegen und ineinandergreifen.

7. Verwendung nach einem der Ansprüche 1 bis 6, **dadurch gekennzeichnet, dass** die beiden Kammern (5, 6) mit jeweils übereinstimmendem Volumen ausgerüstet sind.

8. Verwendung nach einem der Ansprüche 1 bis 7, **dadurch gekennzeichnet, dass** die beiden Kammern (5, 6) in Aufsicht auf die Hülle (4a, 4b) eine im Wesentlichen übereinstimmende Flächenbelegung besitzen.

9. Verwendung nach einem der Ansprüche 1 bis 8, **dadurch gekennzeichnet, dass** die beiden Kammern (5, 6) eine Flächenineinanderschachtelung derart aufweisen, dass jede Teilfläche (9) vorgegebener Größe als Bestandteil einer Gesamtfläche des Wärmekissens (1) eine größtenteils übereinstimmende Flächenbelegung von einerseits der einen Kammer (5) und andererseits der anderen Kammer (6) aufweist.

10. Verwendung nach einem der Ansprüche 1 bis 9, **dadurch gekennzeichnet, dass** sich die Hülle (4a, 4b) aus der oberen Materialbahn (4a) und der unteren Materialbahn (4b) zusammensetzt, welche im Randbereich (7) miteinander verschweißt sind.

11. Verwendung nach Anspruch 10, **dadurch gekennzeichnet, dass** die Schweißverbindung im Randbereich (7) und die Schweißverbindung zur Herstellung der Trennwand (10) zeitgleich in einem Zug definiert werden.

12. Verwendung nach einem der Ansprüche 1 bis 11, **dadurch gekennzeichnet, dass** die beiden Materialbahnen (4a, 4b) aus einem thermoplastischen Kunststoff, beispielsweise PE, PP etc, hergestellt sind.

13. Verwendung nach einem der Ansprüche 1 bis 12, **dadurch gekennzeichnet, dass** das Wärmekissen (1) transparent ausgebildet ist.

14. Verwendung nach einem der Ansprüche 1 bis 13, **dadurch gekennzeichnet, dass** ein das Wärmekissen (1) umhüllender Bezug (12, 13, 14) aus beispielsweise einem Textilmaterial und/oder Schaumstoff vorgesehen ist.

15. Verwendung nach Anspruch 14, **dadurch gekennzeichnet, dass** eine Unterseite des Wärmekissens (1) mit einer wärmeisolierenden Auflage (13, 14) ausgerüstet ist, während eine Oberseite (12) eine die Wärmeleitung nicht oder kaum behindernde Auflage aufweist.
